(19) Europäisches Patentamt
European Patent Office
Office européen des brevets

(11) Veröffentlichungsnummer: **0 042 093**
**A2**

(12) # EUROPÄISCHE PATENTANMELDUNG

(21) Anmeldenummer: **81104164.9**

(22) Anmeldetag: **01.06.81**

(51) Int. Cl.³: **C 07 D 211/90**, A 61 K 31/435

(30) Priorität: **12.06.80 DE 3021958**

(43) Veröffentlichungstag der Anmeldung: **23.12.81**
**Patentblatt 81/51**

(84) Benannte Vertragsstaaten: **AT BE CH DE FR GB IT LI NL SE**

(71) Anmelder: **BAYER AG, Zentralbereich Patente, Marken und Lizenzen, D-5090 Leverkusen 1, Bayerwerk (DE)**

(72) Erfinder: **Materne, Carsten, Dr.,**
**Hans-Böckler-Strasse 31, D-5300 Bonn 3 (DE)**

(54) **4-Aralkyl-1,4-dihydropyridine, Verfahren zu deren Herstellung, ihre Verwendung in Arzneimitteln sowie deren Herstellung.**

(57) Die Erfindung betrifft neue 4-Aralkyl-1,4-dihydropyridine, mehrere Verfahren zu ihrer Herstellung, ihre Verwendung als Arzneimittel, insbesondere zur Beeinflussung von Kreislauferkrankungen, diese enthaltende Arzneimittel, sowie deren Herstellung.

EP 0 042 093 A2

0042093

BAYER AKTIENGESELLSCHAFT 5090 Leverkusen, Bayerwerk

Zentralbereich
Patente, Marken und Lizenzen KS-by-c
Ib (Pha)

# 4-Aralkyl-1,4-dihydropyridine, Verfahren zu deren Herstellung, ihre Verwendung in Arzneimitteln sowie deren Herstellung

Es ist bereits bekannt, daß 1,4-Dihydropyridinderivate Kreislauf-beeinflussende Eigenschaften aufweisen. So ist z.B. der 2,6-Dimethyl-4-(2'-nitrophenyl)-1,4-dihydropyridin-3,5-dicarbonsäuredimethylester (Nifedipin, vgl. DT-PS 16 07 827) als Verbindung bekannt, die coronarvasodilatierende Wirkung besitzt.

1,4-Dihydropyridine, die in 4-Stellung einen Arylalkyl- oder Aryloxyalkylrest tragen, sind bisher nicht beschrieben. Die Erfindung betrifft daher neue 4-Aralkyl-1,4-dihydropyridine der allgemeinen Formel I

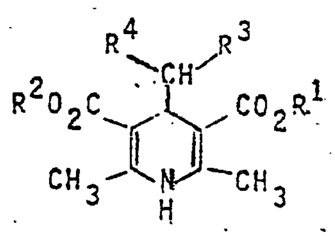

(I)

in der

$R^1$ und $R^2$ gleich oder verschieden sind und einen geradkettigen oder verzweigten Alkylrest mit 1-4 C-atomen bedeuten,

$R^3$ für H, Methyl oder einen Phenylrest und

$R^4$ für einen Phenyl- oder Phenoxyrest steht,

die gegebenenfalls 1 oder mehrere Substituenten aus der Gruppe Nitro, Halogen, Methyl oder Methoxy tragen.

Die neuen Verbindungen besitzen Kreislauf-beeinflussende Eigenschaften, insbesondere erweitern sie die Coronargefäße und senken den Blutdruck.

Die Herstellung der neuen Verbindungen kann in an sich bekannter Weise dadurch erfolgen, daß man

A. einen Aldehyd der Formel II

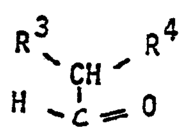

(II)

in der

$R^3$ und $R^4$ die oben angegebene Bedeutung besitzt,

mit Ammoniak und einem Acetessigester der Formel III

$$CH_3-\overset{O}{\overset{\|}{C}}-CH_2-\overset{O}{\overset{\|}{C}}-OR^1 \qquad (III)$$

in der

$R^1$ die oben angegebene Bedeutung besitzt

oder mit einem Enaminoester der Formel IV

$$CH_3-\underset{NH_2}{\underset{|}{C}}=CH-COOR^1 \qquad (IV)$$

in der

$R^1$ die oben angegebene Bedeutung besitzt,

in einem organischen Lösungsmittel umsetzt,

oder

B. Yliden-Verbindungen der Formel V

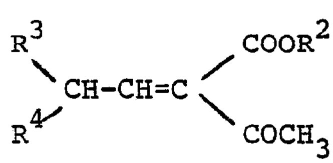

(V)

in der

$R^2, R^3, R^4$ die oben angegebene Bedeutung besitzen,

mit einem Enaminoester der Formel IV in einem organischen Lösungsmittel umsetzt.

Als organisches Lösungsmittel kann Alkohol, Dioxan, Eisesig, Essigester, Dimethylformamid oder Acetonitril genommen werden. Die Reaktionstemperaturen können in einem Bereich zwischen 20°C und 150°C variiert werden. Vorzugsweise arbeitet man bei der Siedetemperatur des Lösungsmittels.

Die verwendbaren Ausgangsstoffe sind meistens bekannte Verbindungen oder sie können nach bekannten Verfahren hergestellt werden.

Als Beispiele für Aldehyde der Formel II seien genannt: 2-(3-Methoxyphenyl)-propionaldehyd, 2-(4'-Methoxyphenyl)-propionaldehyd, 2-(3',4'-Dimethoxyphenyl)-propionaldehyd, 3,4-Dimethoxyphenylacetaldehyd, 2-(4'-Chlorphenyl)-propionaldehyd, 2-(2'-Chlorphenyl)-propionaldehyd, 2-Chlorphenylacetataldehyd, 4-Chlorphenylacetaldehyd, 2-(3-Nitrophenyl)-propionaldehyd, Diphenylacetaldehyd, 2,6-Dimethoxyphenoxyacetaldehyd, 2,6-Dichlorphenoxyacetaldehyd.

Als Acetessigsäureester eignen sich dür diese Umsetzung z.B.: Acetessigsäuremethylester, Acetessigsäureethylester, Acetessigsäurepropylester, Acetessigsäureisopropylester, Acetessigsäurebutylester, Acetessigsäure sec. Butylester, Acetessigsäureisobutylester.

Die für die Umsetzung geeigneten Enaminoester der Formel IV erhält man aus den entsprechenden Acetessigestern der Formel III durch Umsetzen mit Ammoniak.

Die neuen Verbindungen sind als Arzneimittel verwendbare Substanzen. Sie haben ein breites und vielseitiges pharmakologisches Wirkungsspektrum.

Im einzelnen konnten im Tierexperiment folgende Hauptwirkungen nachgewiesen werden:

1) Die Verbindungen bewirken bei parenteraler, oraler und perlingualer Zugabe eine deutliche und langanhaltende Erweiterung der Coronargefäße. Diese Wirkung auf die Coronargefäße wird durch einen gleichzeitigen Nitrit-ähnlichen herzentlastenden Effekt verstärkt.

   Sie beeinflussen bzw. verändern den Herzstoffwechsel im Sinne einer Energieersparnis.

2) Die Erregbarkeit des Reizbildungs- und Erregungsleitungssystems innerhalb des Herzens wird herabgesetzt, so daß eine in therapeutischen Dosen nachweisbare Antiflimmerwirkung resultiert.

3) Der Tonus der glatten Muskulatur der Gefäße wird unter der Wirkung der Verbindungen stark vermindert. Diese gefäßspasmolytische Wirkung kann im gesamten Gefäßsystem stattfinden, oder sich mehr oder weniger isoliert in umschriebenen Gefäßgebieten (wie z.B. dem Zentralnervensystem) manifestieren.

4) Die Verbindungen senken den Blutdruck von normotonen und hypertonen Tieren und können somit als antihypertensive Mittel verwendet werden.

5) Die Verbindungen haben stark muskulär-spasmolytische Wirkungen, dies an der glatten Muskulatur des Magens, Darmtraktes, des Urogenitaltraktes und des Respirationssystems deutlich werden.

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht-toxischer pharmazeutisch geeigneter Trägerstoffe oder Lösungsmittel. Hierbei soll die therapeutusch wirksame Verbindung jeweils in einer Konzentration von etwa 0,5 bis 90 Gew.-% der Gesamtmischung vorhanden sein, d.h. in Mengen, die ausreichens sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielhaft aufgeführt:

Wasser nichttoxische organische Lösungsmittel, wie Paraffine (z.B. Erdölfraktionen), pflanzliche Öle (z.B. Erdnuß-/Sesamöl), Alkohole (z.B. Ethylalkohol, Glycerin), Glykole (z.B. Propylenglykol, Polyethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Roh-, Milch- und Traubenzucker), Emulgiermittel, wie nichtionogene anionische Emulgatoren (z.B. Polyoxyethylen-Fettsäure-Ester, Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspension und/oder Elixieren, die für orale Anwendungen gedacht sind, können die Wirkstoffe außer mit den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,01 bis 10 mg/kg vorzugsweise etwa 0,05 bis 5 mg/kg Körpergewicht pro Tag zur Erzielung wirksamer Ergebnisse zu verabreichen, und bei oraler Applikation beträgt die Dosierung etwa 0,05 bis 20 mg/kg, vorzugsweise 0,5 bis 5 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch auf Grund der Tierart und deren individuellem Verhalten gegenüber dem Medikament bzw. der Art von dessen Formulierung und dem Zeitpunkt bzw. Intervall zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

Im folgenden sei die Erfindung anhand einiger Beispiele zur Herstellung der neuen Dihydropyridin-Derivate erläutert:

Beispiel 1

2,6-Dimethyl-4-/1-(3-methoxyphenyl)-ethyl/-1,4-dihydropyridin-3,5-dicarbonsäuredimethylester

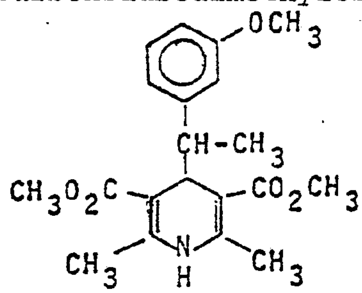

16,4 g 2-(3-Methoxyphenyl)-propionaldehyd, 23,2 g Acetessigsäuremethylester und 2,1 ml $NH_2$ (33 %ig) werden in 50 cm³ Ethanol 4 Stunden unter Rückfluß erhitzt. Nach dem Erkalten kristallisiert das Produkt aus. Zur Reinigung wird aus Ethanol umkristallisiert.
Ausbeute: 26,7 g (75 %)
Schmelzpunkt: 145-147°C

In analoger Weise werden erhalten:
2,6-Dimethyl-4-/1-(3-methoxyphenyl)ethyl/-1,4-dihydropyridin-3,5-dicarbonsäurediisopropylester
2,6-Dimethyl-4-/1-(3-methoxyphenyl)ethyl/-1,4-dihydropyridin-3,5-dicarbonsäuredibutylester
2,6-Dimethyl-4-/1-(2-chlorphenyl)ethyl/-1,4-dihydropyridin-3,5-dicarbonsäurediethylester
2,6-Dimethyl-4-/1-(2-chlorphenyl)ethyl/-1,4-dihydropyridin-3,5-dicarbonsäuredipropylester
2,6-Dimethyl-4-/1-(3-nitrophenyl)ethyl/-1,4-dihydropyridin-3,5-dicarbonsäuredimethylester

Beispiel 2

2,6-Dimethyl-4-(2'-chlorphenylmethyl)-1,4-dihydropyridin-3,5-dicarbonsäuredimethylester

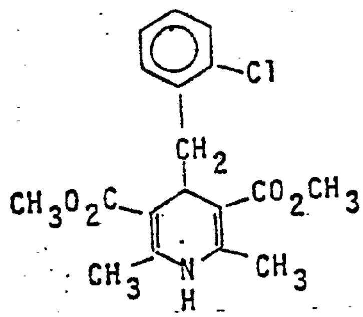

15,4 g 2-Chlorphenylacetaldehyd und 23 g ß-Aminocrotonsäuremethylester werden in 50 ml Ethanol 4 Stunden unter Rückfluß erhitzt. Nach dem Erkalten fällt das Produkt aus. Man kristallisiert aus Methanol um.
Ausbeute: 21 g (60 %)
Schmelzpunkt: 178°C-179°C

In analoger Weise erhält man:
2,6-Dimethyl-4-(2'-chlorphenylmethyl)-1,4-dihydropyridin-3,5-dicarbonsäurediisopropylester
2,6-Dimethyl-4-(3',4'-dimethoxyphenyl)methyl-1,4-dihydropyridin-3,5-dicarbonsäuredimethylester
2,6-Dimethyl-4-(3',4'-dimethoxyphenyl)methyl-1,4-dihydropyridin-3,5-dicarbonsäuredipropylester

Beispiel 3

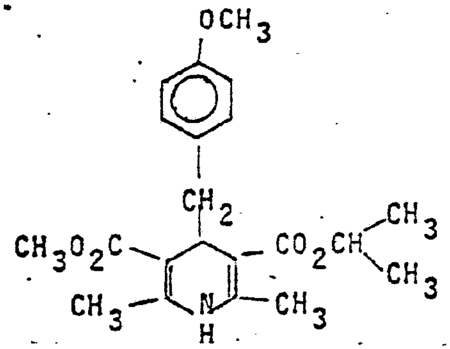

2,6-Dimethyl-4-(4'-methoxyphenylmethyl)-1,4-dihydropyridin-3,5-dicarbonsäure-3-methylester-5-isopropylester.

24,8 g 2-(4'-Methoxyphenyl)ethylidenacetessigsäuremethylester und 14,3 g ß-Aminocrotonsäureisopropylester werden in 100 cm³ Ethanol 4 Stunden unter Rüclfluß erhitzt. Nach dem Erkalten wird das Produkt abgesaugt und aus Ethanol umkristallisiert.
Ausbeute: 18,6 g (50 %)
Schmelzpunkt: 163°C-165°C

In analoger Weise erhält man:
2,6-Dimethyl-4-(3',4'-dimethoxyphenylmethyl)-1,4-dihydropyridin-3,5-dicarbonsäure-3-ethylester-5-isopropylester

Beispiel 4

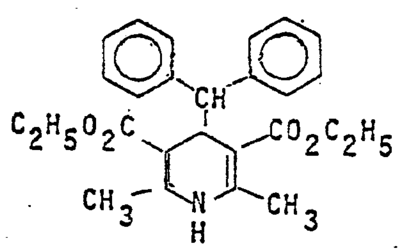

2,6-Dimethyl-4-diphenylmethyl-1,4-dihydropyridin-3,5-dicarbonsäurediethylester

9,6 g Diphenylacetaldehyd und 26 g ß-Aminocrotansäureethylester werden 8 Stunden in 50 $cm^3$ Ethanol unter Rückfluß erhitzt. Nach dem Erkalten fällt das Produkt aus. Man wäscht mit Ether und kristallisiert aus Ethanol um.
Ausbeute: 10,4 g (25 %)
Schmelzpunkt: 140°C

<u>Beispiel 5</u>

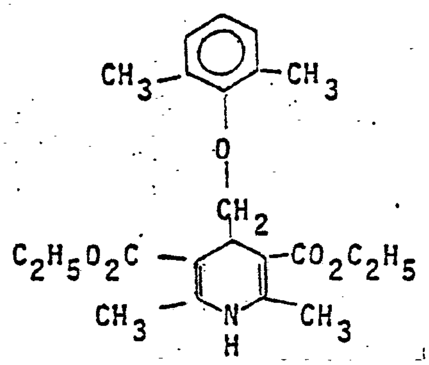

2,6-Dimethyl-4-(2',6'-dimethylphenoxymethyl)-1,4-dihydropyridin-3,5-dicarbonsäurediethylester

16,4 g 2,6-Dimethylphenoxyacetaldehyd und 26 g ß-Aminocrotonsäureethylester werden in 50 $cm^3$ Ethanol 4 Stunden unter Rückfluß erhitzt. Nach dem Erkalten fällt das Produkt aus und wird aus Ethanol umkristallisiert.
Ausbeute: 31,3 g (55 %)
Schmelzpunkt: 106°C-107°C

In analoger Weise erhält man:
2,6-Dimethyl-4-(2',6'-dichlorphenoxymethyl)-1,4-dihydropyridin-3,5-dicarbonsäuredimethylester

Patentansprüche

1. 4-Aralkyl-1,4-dihydropyridine der allgemeinen Formel I

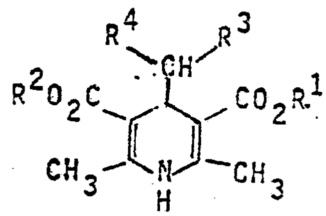

(I)

in der

$R^1$ und $R^2$ gleich oder verschieden sind und einen geradkettigen oder verzweigten Alkylrest mit 1-4 C-Atomen bedeuten,

$R^3$ für H, Methyl oder einen Phenylrest und

$R^4$ für einen Phenyl- oder Phenoxyrest steht,

die gegebenenfalls 1 oder mehrere Substituenten aus der Gruppe Nitro, Halogen, Methyl oder Methoxy tragen.

2. Verfahren zur Herstellung der 1,4-Dihydropyridinderivate nach Anspruch 1, dadurch gekennzeichnet, daß man in an sich bekannter Weise

A. einen Aldehyd der Formel II

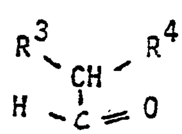

(II)

in der

$R^3$ und $R^4$ die oben angegebene Bedeutung besitzt,

mit Ammoniak und einem Acetessigester der Formel III

$$CH_3-\overset{\overset{O}{\parallel}}{C}-CH_2-\overset{\overset{O}{\parallel}}{C}-OR^1 \qquad \text{(III)}$$

in der

$R^1$ die oben angegebene Bedeutung besitzt

oder mit einem Enaminoester der Formel IV

$$CH_3-\underset{\underset{NH_2}{|}}{C}=CH-COOR^1 \qquad \text{(IV)}$$

in der

$R^1$ die oben angegebene Bedeutung besitzt,

in einem organischen Lösungsmittel umsetzt, oder

B. Yliden-Verbindungen der Formel V

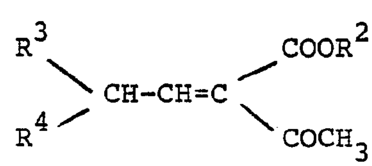

(V)

in der

$R^2, R^3, R^4$ die oben angegebene Bedeutung besitzen,

mit einem Enaminoester der Formel IV in einem organischen Lösungsmittel umsetzt.

3. Arzneimittel, gekennzeichnet durch einen Gehalt an 1,4-Dihydropyridin-Derivaten gemäß Anspruch 1 und üblichen pharmazeutischen Hilfsstoffen.

4. Verfahren zur Herstellung von Arzneimitteln, dadurch gekennzeichnet, daß man Verbindungen der allgemeinen Formel I gemäß Anspruch 1 gegebenenfalls unter Verwendung von Hilfs- und Trägerstoffen in eine geeignete Applikationsform überführt.

5. Verwendung von Verbindungen der allgemeinen Formel I gemäß Anspruch 1 bei der Bekämpfung von Krankheiten.

6. Verwendung von Verbindungen der allgemeinen Formel I gemäß Anspruch 1 bei der Bekämpfung von Kreislauferkrankungen.